# EUROPEAN PATENT APPLICATION

(11) **EP 4 803 191 A1**
(43) Date of publication of application: **09.09.2026**
(21) Application number: 24897335.6
(22) Date of filing: 15.11.2024
(51) Int. Cl.: B01J 20/22, B01D 53/14, B01J 20/34, C01B 32/50, C07C 211/27

(54) **SOLID ABSORBER, AND METHOD FOR ABSORBING AND DESORBING ACIDIC GAS BY USING SAME**

(30) Priority: 28.11.2023 JP 2023200852
(71) Applicant: KAWASAKI JUKOGYO KABUSHIKI KAISHA, Kobe-shi, Hyogo 650-8670 (JP)
(72) Inventor: OKUMURA, Takeshi, Kobe-shi, Hyogo 650-8670 (JP); NISHIBE, Shohei, Kobe-shi, Hyogo 650-8670 (JP); NUMAGUCHI, Ryohei, Kobe-shi, Hyogo 650-8670 (JP)
(74) Representative: Arnold & Siedsma
(86) International application number: PCT/JP2024/040611
(87) International publication number: WO 2025/115648

(57) **Abstract**

This solid absorber reversibly absorbs an acidic gas, and comprises a porous base material and an acidic gas absorbing agent supported on the porous base material. The acidic gas absorbing agent contains at least one amine represented by any one of general formulae (1), (2), and (3). (In the formulae (1), (2), and (3), R¹ is represented by a group: -(CH₂)1-NH-[(CH₂)₂NH]ₘ-(CH₂)ₙ-CH₃, and in R¹, 1 represents 1 or 2, and m and n each independently represent an integer of 0 to 2.)

## Description

### Technical Field

The present invention relates to a solid absorber that reversibly absorbs an acidic gas, and a method for absorbing and desorbing an acidic gas using the solid absorber.

### Background Art

Conventionally, there has been known a solid absorber that reversibly absorbs acidic gases such as carbon dioxide, NOx (nitrogen oxide), SOx (sulfur oxide), and HCl contained in combustion exhaust gas discharged from combustion facilities such as a thermal power plant, a blast furnace of a steel plant, a converter of a steel plant, and a boiler. In addition, a technique has also been developed in which such a solid absorber is applied to DAC (Direct Air Capture) that directly captures these acidic gases, particularly carbon dioxide, from the atmosphere or the like. Such a solid absorber is generally formed by making an amine being a liquid chemical substance that reversibly absorbs an acidic gas to be carried on a porous base material (for example, porous material particles).

As an example of such a solid absorber, Patent Literature 1 describes a carbon dioxide separating material comprising a polyamine carrier in which a polyamine having at least two isopropyl groups on a nitrogen atom is carried on a support.

In addition, as another example of such a solid absorber, Patent Literature 2 describes an acidic gas absorber comprising porous material particles and an acidic gas absorbing agent (for example, amine) carried on the porous material particles. In the acidic gas absorber described in Patent Literature 2, the porous material particles have binary pores including a mesopore having a pore diameter in a nanometer region of 2 nm or more and 200 nm or less and a macropore having a pore diameter in a micrometer region of more than 0.2 µm, wherein the macropore is an empty pore, and the mesopore is filled with the acidic gas absorbing agent.

### Citation List

### Patent Literature

Patent Literature 1: JP 2015-9185 A
Patent Literature 2: JP 2020-75215 A

### Summary of Invention

An object of the present disclosure is to provide a solid absorber that has low hygroscopicity and is capable of efficiently and reversibly absorbing an acidic gas, particularly carbon dioxide.

A solid absorber according to a first aspect of the present disclosure is a solid absorber that reversibly absorbs an acidic gas,
the solid absorber comprising a porous base material and an acidic gas absorbing agent carried on the porous base material
wherein the acidic gas absorbing agent comprises at least one amine represented by any one of the following general formulae (1), (2), and (3): 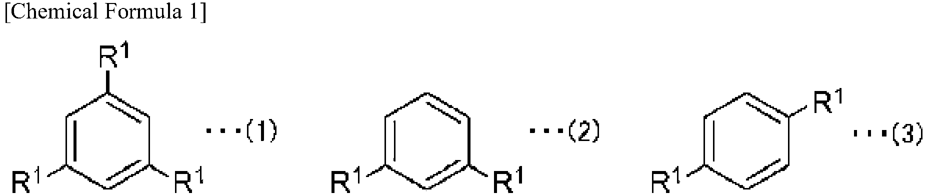
in the formulae (1), (2), and (3), R¹ is represented by group: -(CH₂)ₗ-NH-[(CH₂)₂NH]ₘ-(CH₂)ₙ-CH₃, and in R¹, l represents 1 or 2, and m and n each independently represent an integer of 0 to 2.

Alternatively, another solid absorber according to the first aspect of the present disclosure comprises a porous base material and an acidic gas absorbing agent carried on the porous base material,
wherein the acidic gas absorbing agent comprises at least one amine represented by the following general formula (4): 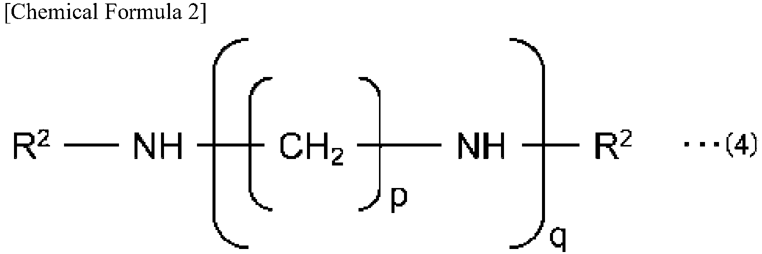
in the formula (4), two R² groups may have the same structure or different structures, p represents 2 or 3, q represents an integer of 2 to 5, and R² is a group represented by any one of the following R²(1) to R²(9).

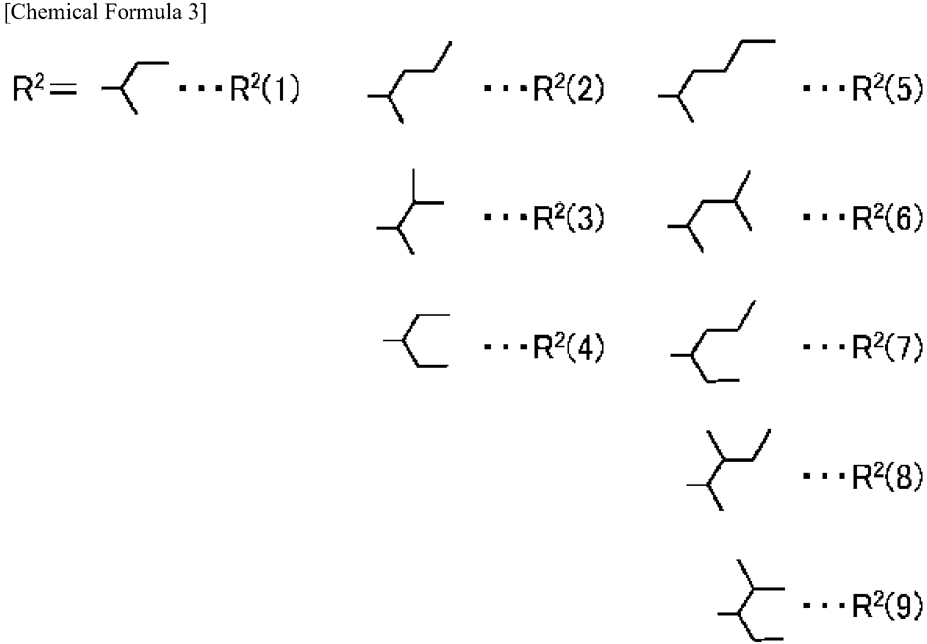

A method for absorbing and desorbing an acidic gas according to a second aspect of the present disclosure comprises:
a step of bringing a gas to be treated into contact with any one solid absorber of a solid absorber according to the first aspect and another solid absorber according to the first aspect to cause the solid absorber to absorb the acidic gas, and
a step of bringing a vapor into contact with the solid absorber having absorbed the acidic gas to cause the acidic gas to be desorbed from the solid absorber.

### Effects of the Invention

According to the present disclosure, it is possible to provide a solid absorber that has low hygroscopicity and is capable of efficiently and reversibly absorbing an acidic gas, particularly carbon dioxide.

### Brief Description of Drawings

[FIG. 1] FIG. 1 is a diagram showing existence positions a to e of hydrogen in an amine represented by (1-1), which is a target product of Example 1, and the measured 1H-NMR chart.
[FIG. 2] FIG.2 is a diagram showing existence positions a to e of hydrogen in an amine represented by (2-1), which is a target product of Example 2, and the measured 1H-NMR chart.
[FIG. 3] FIG. 3 is a diagram showing existence positions a to c of hydrogen in an amine represented by (3-1), which is a target product of Example 3, and the measured 1H-NMR chart.
[ FIG. 4] FIG. 4 is a diagram showing existence positions a to g of hydrogen in an amine represented by (4-4), which is a target product of Example 4, and the measured 1H-NMR chart.
[FIG. 5] FIG. 5 is a diagram showing a 1H-NMR chart measured in Example 5.
[FIG. 6] FIH. 6 is a diagram showing existence positions a to i of hydrogen in an amine represented by (4-3), which is a target product of Example 6, and the measured 1H-NMR chartd.
[FIG. 7] FIG. 7 is a graph showing a measurement result of a saturated carbon dioxide absorption amount (mol/kg) per weight of the solid absorber in each of the Examples.
[FIG. 8] FIG. 8 is a graph showing a measurement result of a saturated moisture absorption amount (mol/kg) per weight of the solid absorber in each of the Examples.

### Description of Embodiments

A solid absorber that reversibly absorbs an acidic gas like that described above is generally utilized in an acidic gas separation system (particularly a carbon dioxide separation system). Examples of the acidic gas separation system include systems of a batch processing type, a moving bed type (a continuous processing type), and the like. In these acidic gas separation systems, a step of causing an acidic gas to be absorbed to a solid absorber and a step of causing the acidic gas to be desorbed from the solid absorber using vapor or the like (that is, a step of regenerating the solid absorber) are repeatedly performed. Therefore, the solid absorber is required to efficiently absorb and desorb an acidic gas, particularly carbon dioxide.

In addition, in such an acidic gas separation system, where the solid absorber has excessively absorbed moisture, the solid absorber exhibits stickiness, and ease in handling thereof is deteriorated. When ease in handling of the solid absorber is poor, in a system of a moving bed type involving movement of the solid absorber, the acidic gas absorption characteristics of the solid absorber are significantly deteriorated. Even in a system of a batch processing type, when ease in handling of the solid absorber is poor, it is difficult to replace the solid absorber. Therefore, the solid absorber preferably has low hygroscopicity.

The present inventors have conducted various studies on a solid absorber that has low hygroscopicity and is capable of efficiently and reversibly absorbing an acidic gas, particularly carbon dioxide. Then, it has been found that such an effect can be exhibited when, in a solid absorber comprising a porous base material and an acidic gas absorbing agent carried on the porous base material, the acidic gas absorbing agent comprises at least one amine represented by a prescribed general formula. In addition, it has also been found that the solid absorber can desorb an acidic gas using exhaust heat, which is generally not effectively usable.

In the present description, the "exhaust heat" means low-temperature heat energy at 40°C or more and 100°C or less that is unavoidably generated from various manufacturing industries, power plants, and the like, which cannot be effectively used generally, sunlight-derived low-temperature heat energy, which cannot be effectively used generally, and the like. Therefore, the solid absorber in the present embodiment is superior also in an environmental aspect.

In the present description, the "acidic gas" generally means an acidic gases such as carbon dioxide, NOx (nitrogen oxides such as NO, NO₂, N₂O, and N₂O₃), SOx (sulfur oxides), and HCl, which are contained in combustion exhaust gas, the atmosphere, a closed space atmosphere, and the like. Among them, the acidic gas to be absorbed is preferably carbon dioxide from the viewpoint of exhibiting a good effect in an environmental aspect.

In the following, embodiments of the present invention will be described in detail with reference to the drawings. The scope of the present invention is not limited to the embodiment described herein, and various modifications can be made unless the gist of the present invention is impaired.

### 1. Solid Absorber

The solid absorber in the present embodiment will be described in detail below. In the present description, the "solid absorber" means a solid absorber capable of reversibly absorbing an acidic gas. In addition, in the present description, "capable of reversibly absorbing an acidic gas (particularly carbon dioxide)" means capable of absorbing (or recovering) the acidic gas (particularly carbon dioxide) and desorbing the acidic gas (particularly carbon dioxide) (in other words, regenerating the solid absorber). The solid absorber in the present embodiment includes a first solid absorber and a second solid absorber.

### [First Solid Absorber]

The first solid absorber comprises a porous base material and an acidic gas absorbing agent carried on the porous base material. First, the acidic gas absorbing agent in the first solid absorber will be described in detail below.

### (Acidic Gas Absorbing Agent)

The acidic gas absorbing agent in the first solid absorber comprises at least one amine represented by any one of the following general formulae (1), (2), and (3) (hereinafter, also referred to as "amine of the first solid absorber").

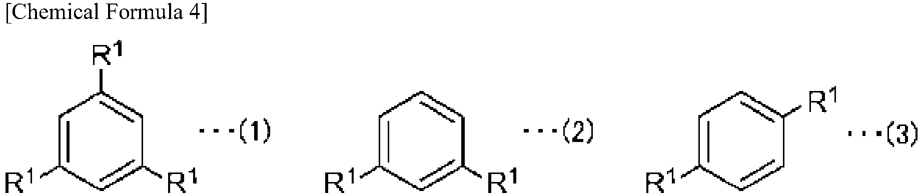

In the formulae (1), (2), and (3), R¹ is represented by group: -(CH₂)ₗ-NH-[(CH₂)₂NH]ₘ-(CH₂)ₙ-CH₃, and in R¹, l represents 1 or 2, and m and n each independently represent an integer of 0 to 2.

In R¹, l is preferably 1. When l is 1, the amine of the first solid absorber that has a remarkably stable structure can be obtained in a high yield, and finally the production efficiency of the solid absorber can be improved.

In R¹, m preferably represents 0 or 1. When m is 0 or 1, finally, a solid absorber having good low-hygroscopicity characteristics and being capable of more efficiently desorbing an acidic gas, particularly carbon dioxide can be produced.

In R¹, n preferably represents 0 or 1, and more preferably represents 0. When n is 0, finally, a solid absorber capable of more efficiently and reversibly absorbing an acidic gas, particularly carbon dioxide can be produced.

The acidic gas absorbing agent in the first solid absorber may be a mixture of two or more of the amines represented by any one of the general formulae (1), (2) and (3), may comprise a single amine among them, or may be a single amine among them.

More specifically, the amine of the first solid absorber preferably has a structure represented by any one of the following (1-1), (1-2), (2-1), and (3-1).

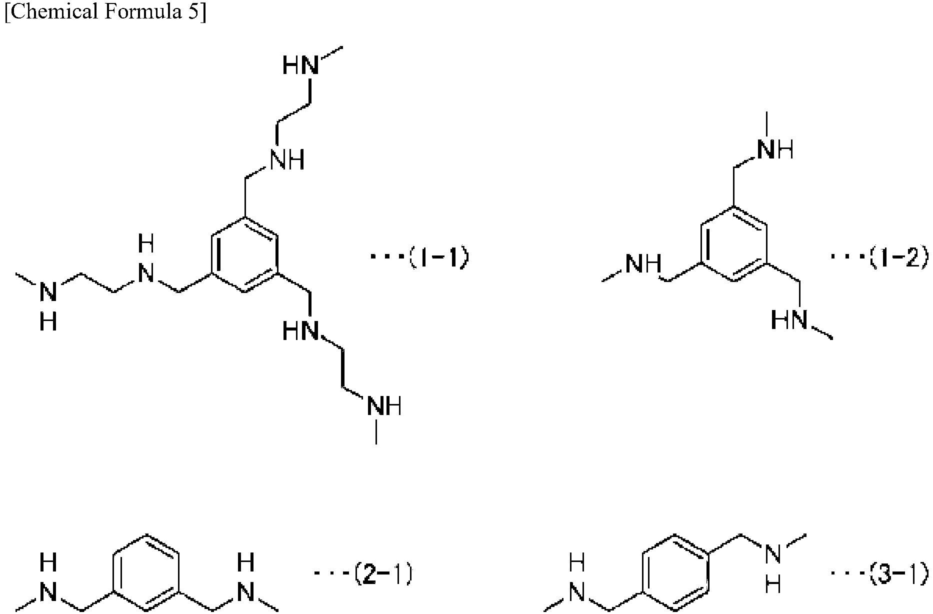

When the amine of the first solid absorber has the structure represented by any one of the above, finally, a solid absorber having good low-hygroscopicity and being capable of more efficiently and reversibly absorbing an acidic gas, particularly carbon dioxide can be produced.

The method for synthesizing the amine of the first solid absorber is not particularly limited, and the amine may be synthesized by any method known to those skilled in the art. For example, the amine of the first solid absorber can be synthesized by the following method. Specifically, the amine of the first solid absorber can be synthesized by introducing a modification chain having an amine by a reductive amination reaction using a benzaldehyde having a plurality of formyl groups as a starting substance.

More specifically, for example, the amine of the first solid absorber having a structure represented by any one of the above (1-1), (2-1), and (3-1) can be synthesized by the method described in detail in the following Examples.

The content of the amine in the first solid absorber based on the total mass of the first solid absorber is not particularly limited, but is preferably 10 mass% or more. When the content of the amine in the first solid absorber is 25 mass% or more, the solid absorber finally produced can more efficiently absorb and desorb an acidic gas, particularly carbon dioxide. The content of the amine in the first solid absorber is more preferably 10 mass% or more, and still more preferably 25 mass% or more. The upper limit of the content of the amine in the first solid absorber is not particularly limited, but is, for example, 50 mass% or less.

The acidic gas absorbing agent may be composed of only the at least one amine of the first solid absorber described above, but may contain another compound as long as the effect of the first solid absorber in the present embodiment is exhibited. For example, the acidic gas absorbing agent may contain an amine compound other than the amine of the first solid absorber, polyethyleneimine, tetraethylenepentamine, an amine-based or non-amine-based byproduct (inevitably) contained at the time of amine synthesis, or the like.

### (Porous Base Material)

The porous base material carries the above-described acidic gas absorbing agent thereon.

The type of the porous base material is not particularly limited. Specifically, as the porous base material, a base material made of any material known to those skilled in the art, the material being capable of carrying the above-described acidic gas absorbing agent thereon and having a large number of pores available for reversibly absorbing an acidic gas, particularly carbon dioxide, can be used.

Examples of the porous base material include silica such as silica gel or mesoporous silica, alumina such as activated alumina, zeolite, titania, zirconia, magnesia, activated carbon, and metal-organic frameworks (MOF). A powder may be used as the porous base material. The porous base material may be molded using a powder thereof and a binder as appropriate, and for example, may be made granular (typically 1 mm to 5 mm) at the time of producing the porous base material.

### (Method for Producing Solid Absorber)

The first solid absorber comprises a porous base material and an acidic gas absorbing agent carried on the porous base material. A method for producing the first solid absorber will be described below.

### (1) Step of Preparing Acidic Gas Absorbing Agent Solution

First, the at least one amine of the first solid absorber described above contained in the acidic gas absorbing agent is dissolved in a solvent (water or alcohol) to prepare an acidic gas absorbing agent solution.

The temperature of the prepared acidic gas absorbing agent solution containing the amine of the first solid absorber is not particularly limited, but is preferably 10°C or higher and 100°C or lower. Since it is known that the viscosity of a solution containing an amine decreases with an increase in temperature, when the temperature of the acidic gas absorbing agent solution is 10°C or higher, the amine can be uniformly carried on the porous base material. When the temperature of the acidic gas absorbing agent solution is 100°C or lower, oxidation and/or evaporation of the amine can be inhibited.

The concentration of the amine of the first solid absorber in the acidic gas absorbing agent solution is not particularly limited, but may be adjusted to an appropriate value in the range of 5 mass% or more and 70 mass% or less according to the type of the amine of the first solid absorber. If the concentration of the amine of the first solid absorber in the solution is excessively low, the amount of the amine carried on the porous base material may be insufficient. In addition, if the concentration of the amine of the first solid absorber in the solution is excessively high, the amine may block the pores of the porous base material, leading to deterioration of the acidic gas absorption performance of the first solid absorber.

### (2) Impregnation Step

Next, the porous base material (for example, the above-described porous material particles) is charged into an immersion container filled with the acidic gas absorbing agent solution, and the porous base material is impregnated with the acidic gas absorbing agent solution. The immersion time of the porous base material is not particularly limited as long as the time is long enough for sufficiently degassing the inside of the pores. For example, the immersion time may be 24 hours. At this time, to shorten the immersion time, the acidic gas absorbing agent solution may be stirred or ultrasonic vibration may be applied to the immersion container.

### (3) Drying Step

Thereafter, the porous base material is pulled up from the acidic gas absorbing agent solution, and the attached excess liquid is removed by such a method as suction filtration. Further, thereafter, the porous base material impregnated with the acidic gas absorbing agent solution is dried by through-flow drying or drying under reduced pressure, at a temperature near room temperature. After drying, a first solid absorber can be obtained.

### [Second Solid Absorber]

The second solid absorber also comprises a porous base material and an acidic gas absorbing agent carried on the porous base material. First, the acidic gas absorbing agent in the second solid absorber will be described in detail below.

### (Acidic Gas Absorbing Agent)

The acidic gas absorbing agent in the second solid absorber comprises at least one amine represented by the following general formula (4) (hereinafter, also referred to as "amine of the second solid absorber").

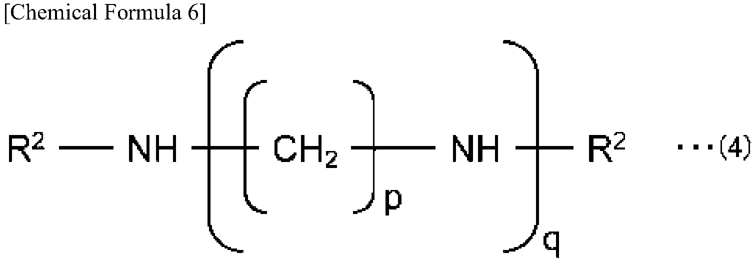

In the formula (4), two R² groups may have the same structure or different structures, p represents 2 or 3, q represents an integer of 2 to 5, and R² is a group represented by any one of the following R²(1) to R²(9).

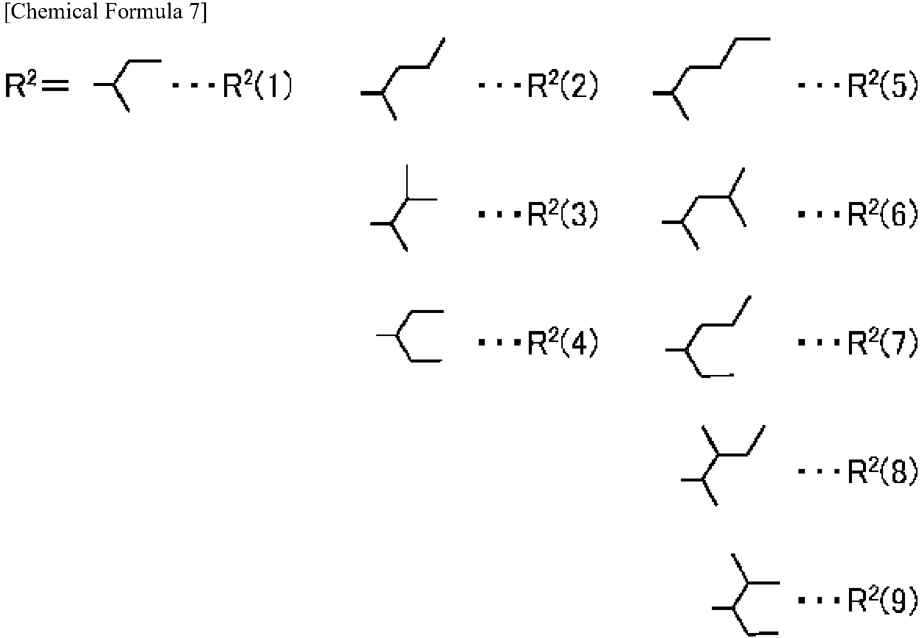

In the present description, the phrase "two R² groups may have the same structure or different structures" means that the two R² groups located at the ends in the general formula (4) may be the same group represented by any of the R²(1) to R²(9), or may be groups different from each other represented by any of R²(1) to R²(9).

p is preferably 3. When p is 3, finally, a solid absorber having good low-hygroscopicity characteristics and being capable of more efficiently desorbing an acidic gas, particularly carbon dioxide can be produced.

q preferably represents 2 or 3, and more preferably represents 2. When q is 2, finally, a solid absorber having good low-hygroscopicity and being capable of more efficiently and reversibly absorbing an acidic gas, particularly carbon dioxide can be produced.

The acidic gas absorbing agent in the second solid absorber may be a mixture of two or more amines among the amines represented by the general formula (4), may contain a single amine among them, or may be a single amine among them.

More specifically, the amine of the second solid absorber preferably has a structure represented by any one of the following (4-1) to (4-6).

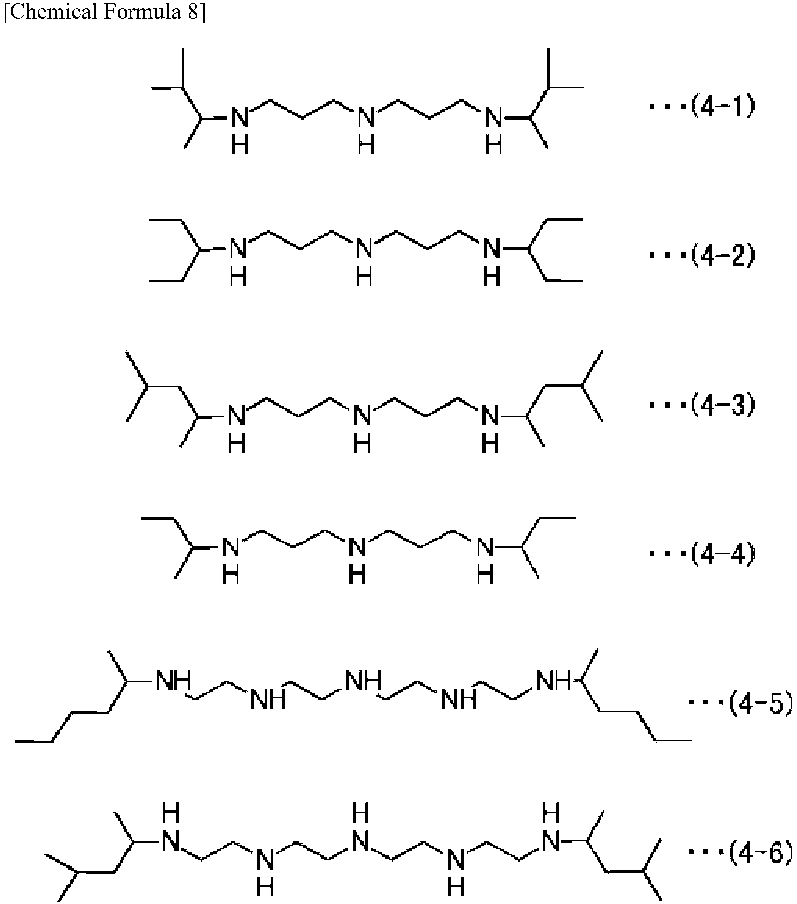

When the amine of the second solid absorber has the structure represented by any one of the above, finally, a solid absorber having good low-hygroscopicity and being capable of more efficiently and reversibly absorbing an acidic gas, particularly carbon dioxide can be produced.

Further, the amine of the second solid absorber more preferably has a structure represented by any one of the above (4-1) to (4-3). When the amine of the second solid absorber has the structure represented by any one of the above (4-1) to (4-3), finally, a solid absorber having good low-hygroscopicity and being capable of more efficiently and reversibly absorbing an acidic gas, particularly carbon dioxide can be more reliably produced.

The method for synthesizing the amine of the second solid absorber is not particularly limited, and the amine may be synthesized by any method known to those skilled in the art. For example, the amine of the second solid absorber can be synthesized by the following method. First, a chain alkyl group is introduced into a commercially available polyamine having a group: - NH or a polyamine having a group: -NH obtained by any method known to those skilled in the art, by a reaction known to those skilled in the art, for example, by an addition reaction of an alkyl halide or a reductive amination of a ketone with a terminal amino group.

More specifically, for example, the amine of the second solid absorber having a structure represented by any one of the above (4-1), (4-2), and (4-3) can be synthesized by the method described in detail in the following Examples.

The preferred amount of the content of the amine of the second solid absorber based on the total mass of the second solid absorber is the same as the preferred amount of the content of the amine of the first solid absorber based on the total mass of the first solid absorber.

The acidic gas absorbing agent may be composed of only the at least one amine of the second solid absorber described above, but may contain another compound as long as the effect of the second solid absorber in the present embodiment is exhibited, as in the case of the first solid absorber described above.

The details of the porous base material in the second solid absorber and the method for producing the solid absorber are also the same as those of the first solid absorber described above.

As described above, the first and second solid absorbers in the present embodiment have low hygroscopicity characteristics, and are capable of efficiently and reversibly absorbing an acidic gas, particularly carbon dioxide. Further, the first and second solid absorbers in the present embodiment can desorb an acidic gas from the solid absorbers by using exhaust heat that generally cannot be effectively utilized, and thus are superior also in an environmental aspect.

### 2. Method for Absorbing and Desorbing Acidic Gas

The method for absorbing and desorbing an acidic gas (particularly carbon dioxide) in the present embodiment includes a step of bringing a gas to be treated into contact with the first or second solid absorber in the above-described embodiment to cause the solid absorber to absorb the acidic gas, and a step of bringing steam into contact with the solid absorber having absorbed the acidic gas to cause the acidic gas to be desorbed from the solid absorber.

In the present description, the "gas to be treated" is not particularly limited as long as it is a gas containing an acidic gas, particularly a gas containing carbon dioxide. Examples of such a gas include combustion exhaust gas discharged from combustion facilities such as a thermal power plant using coal, heavy oil, natural gas, or the like as a fuel, a steel plant's blast furnace that reduces iron oxide with coke, a steel plant's converter that burns carbon in pig iron to make steel, and a boiler in various manufacturing plants, and gas discharged from transportation devices using gasoline, heavy oil, light oil, or the like as a fuel such as automobiles, ships, and aircrafts. In addition, other examples of the gas to be treated include an atmosphere containing an acidic gas, particularly carbon dioxide, and a surrounding atmosphere containing carbon dioxide discharged through human respiration, energy conversion of devices, or the like in a sealed space such as a submersible research vehicle or a space station.

The acidic gas content and the temperature in the gas to be treated are not particularly limited as long as the acidic gas can be absorbed when the first or second solid absorber in the above-described embodiment comes into contact with the acidic gas. For example, when the acidic gas is carbon dioxide, it is sufficient that the carbon dioxide partial pressure is 0.04 kPa to 50 kPa, and the temperature is 20°C to 60°C.

For example, when the acidic gas is carbon dioxide, in the step of desorbing carbon dioxide, carbon dioxide may be desorbed by bringing steam at, for example, 50°C or higher into contact with the solid absorber as in any method known to those skilled in the art. Furthermore, from the viewpoint of efficiently desorbing carbon dioxide, in the step of desorbing carbon dioxide, it is preferable to bring steam into contact under a reduced pressure condition. The reduced pressure condition is preferably 5 kPa or more and 100 kPa or less, and more preferably 15 kPa or more and 30 kPa or less.

On the other hand, as described in the above embodiment, the first and second solid absorbers that have absorbed an acidic gas can desorb the acidic gas even when exhaust heat that generally cannot be utilized effectively is used. Therefore, in the step of desorbing the acidic gas, it is preferable, from the viewpoint of being superior in an environmental aspect, to desorb the acidic gas from the solid absorber by bringing low-temperature steam generated by exhaust heat into contact. For example, when the acidic gas is carbon dioxide, the temperature of the exhaust heat is preferably 40°C or more and 100°C or less, and more preferably 50°C or more and 70°C or less.

By adopting the method in the present embodiment in, for example, a medium-scale or large-scale acidic gas separation system (particularly, a carbon dioxide separation system), a system that is superior also in an environmental aspect and can efficiently absorb and desorb an acidic gas (particularly carbon dioxide) from exhaust gas can be achieved.

### [Summary of the Present Disclosure]

The specific embodiments described above include a disclosure having the following configurations.

A solid absorber according to a first mode of the present disclosure is a solid absorber that reversibly absorbs an acidic gas,
the solid absorber comprising a porous base material and an acidic gas absorbing agent carried on the porous base material
wherein the acidic gas absorbing agent comprises at least one amine represented by any one of the following general formulae (1), (2), and (3): 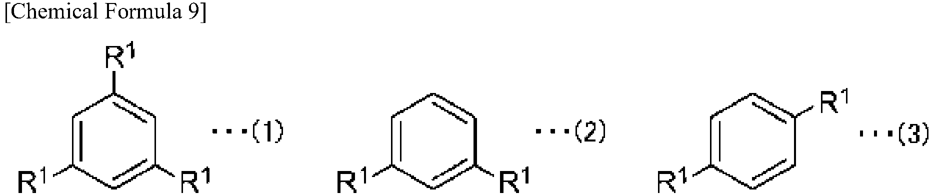
in the formulae (1), (2), and (3), R¹ is represented by group: -(CH₂)ₗ-NH-[(CH₂)₂NH]ₘ-(CH₂)ₙ-CH₃, and in R¹, l represents 1 or 2, and m and n each independently represent an integer of 0 to 2.

The solid absorber of the first mode has low hygroscopicity, and is capable of efficiently and reversibly absorbing an acidic gas, particularly carbon dioxide.

A solid absorber of a second mode of the present disclosure is the solid absorber of the first mode, wherein the amine has a structure represented by any one of the following (1-1), (1-2), (2-1), and (3-1).

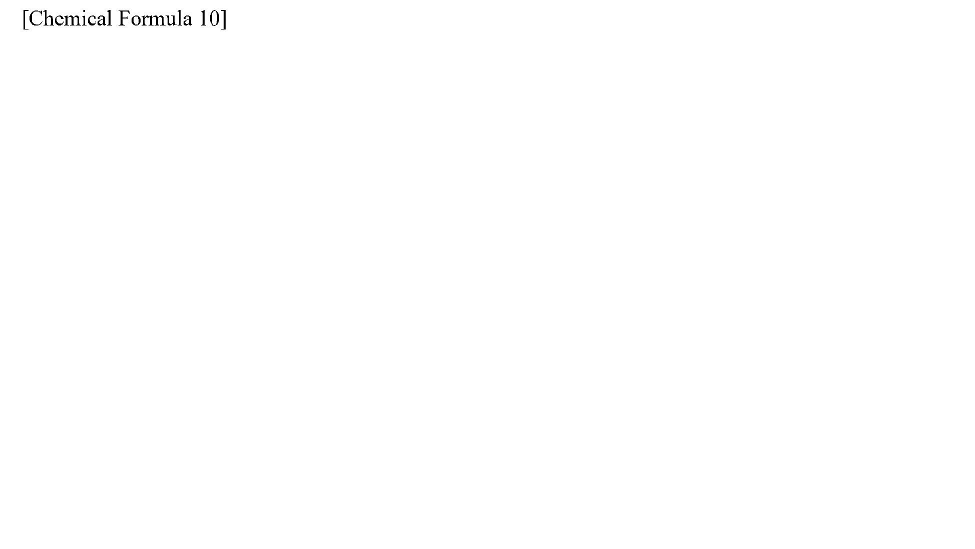

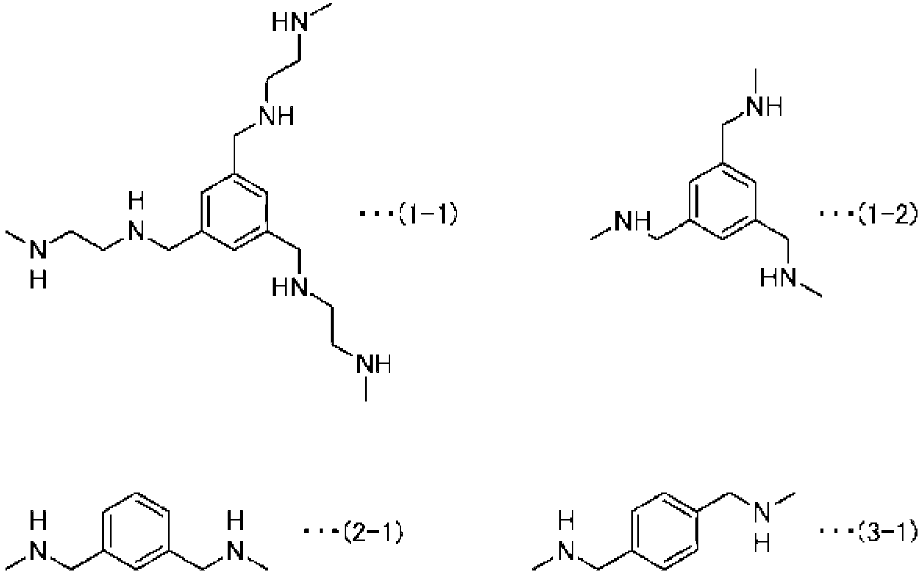

The solid absorber of the second mode has good low-hygroscopicity and is capable of reversibly absorbing an acidic gas, particularly carbon dioxide, more efficiently.

A solid absorber of a third mode of the present disclosure is a solid absorber that reversibly absorbs an acidic gas,
the solid absorber comprising a porous base material and an acidic gas absorbing agent carried on the porous base material
wherein the acidic gas absorbing agent comprises at least one amine represented by the following general formula (4): 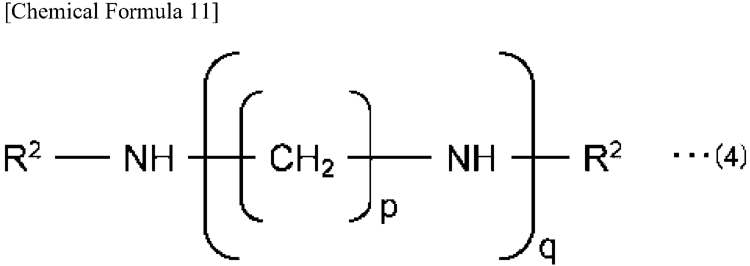
in the formula (4), two R² groups may have the same structure or different structures, p represents 2 or 3, q represents an integer of 2 to 5, and R2 is a group represented by any one of the following R²(1) to R²(9).

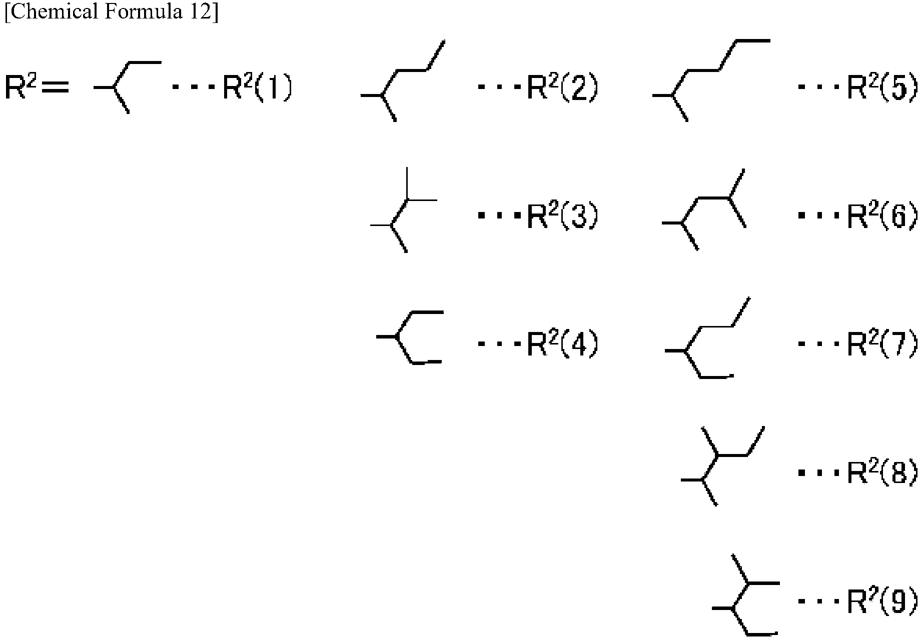

The solid absorber of the third mode has low hygroscopicity, and is capable of efficiently and reversibly absorbing an acidic gas, particularly carbon dioxide.

A solid absorber of a fourth mode of the present disclosure is the solid absorber of the third mode, wherein the amine has a structure represented by any one of the following (4-1) to (4-6).

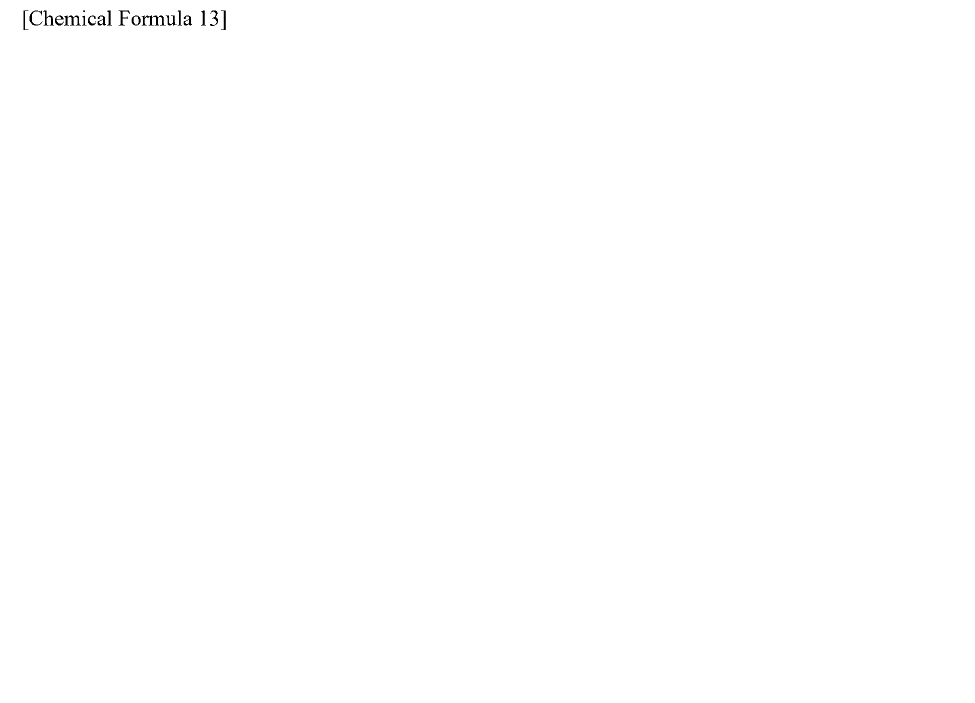

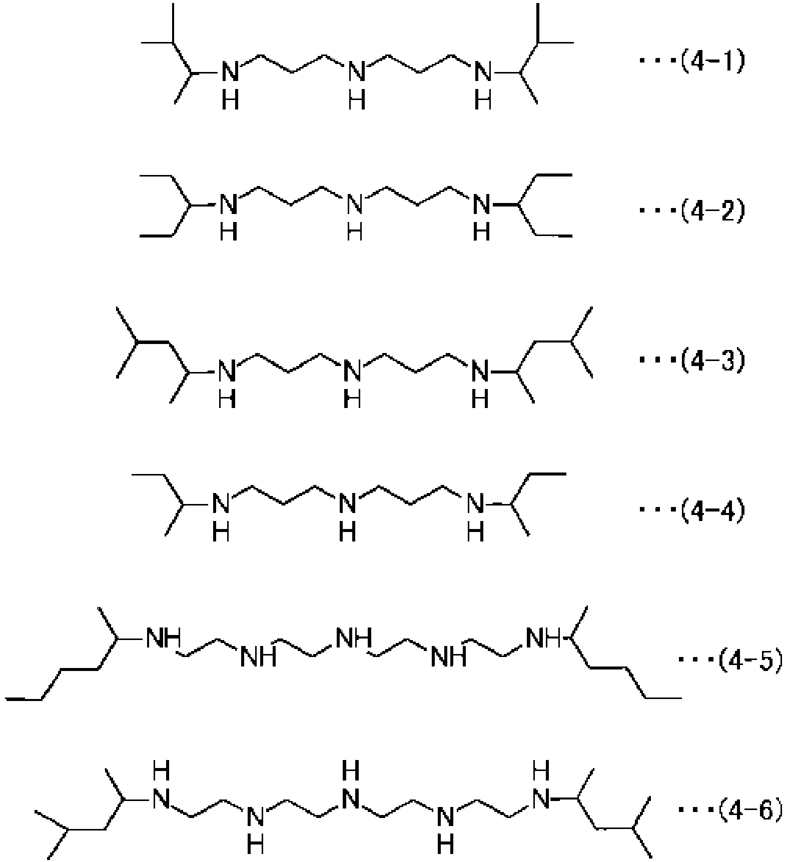

The solid absorber of the fourth mode has good low-hygroscopicity, and is capable of reversibly absorbing an acidic gas, particularly carbon dioxide, more efficiently.

A method for absorbing and desorbing an acidic gas according to a fifth mode of the present disclosure includes:
a step of bringing a gas to be treated into contact with the solid absorber according to any one of the first to fourth modes to cause the solid absorber to absorb the acidic gas, and
a step of bringing steam into contact with the solid absorber having absorbed the acidic gas to cause the acidic gas to be desorbed from the solid absorber.

According to the method for absorbing and desorbing an acidic gas of the fifth mode, since the solid absorber has low hygroscopicity, it is possible to prevent deterioration of the handling performance of the solid absorber in, for example, acidic gas separation systems of a moving bed type and a batch processing type.

### Examples

Hereinafter, the present invention will be explained more specifically with reference to Examples, but the present invention is not limited at all by the Examples.

In the Examples, the first and second solid absorbers in the present embodiment were actually produced, and the saturated carbon dioxide absorption amount (kg/m³) and the saturated moisture absorption amount (kg/m³) of the solid absorbers produced were measured.

First, a method for synthesizing the amine (that is, the acidic gas absorbing agent) of the solid absorber used in each of the Examples and Comparative Example, the porous base material used, and a method for producing the solid absorber will be described in detail below. Incidentally, a proton nuclear magnetic resonance (1H-NMR) apparatus ("AL-400, ECZ400S" manufactured by JEOL Ltd.), a gas chromatography (GC) analyzer ("GC-2010 PlusAF" manufactured by Shimadzu Corporation), and a gas chromatography-mass spectrometry (GCMS) apparatus ("GC/MS QP-2010 Plus" manufactured by Shimadzu Corporation) were used to confirm the target amine except for Example 5.

### [Method for Synthesizing Amine of Solid Absorber]

### (Example 1)

In Example 1, an amine having a structure represented by the above (1-1), which was the amine of the first solid absorber, was used. The synthesis method is described in detail below.

First, 1,3,5-benzenetricarbaldehyde (reference substance) was added to a solution of N-Boc-methylethylenediamine in ethanol, and the solution resulting from the addition was heated and stirred at 90°C for 1.5 hours. Thereafter, the reaction solution was cooled to around 0°C, and a reducing agent was added to reduce an imine. Subsequently, a lipophilic component was collected from the reaction product obtained by reducing the imine, by liquid-liquid extraction separation using chloroform and water. Thereafter, the protective group of the imine was removed by adding trifluoroacetic acid, and from the collected lipophilic component, a target product supposed to be the amine having the structure represented by the above (1-1) was obtained as a yellow oily substance. The yield was 54.6%.

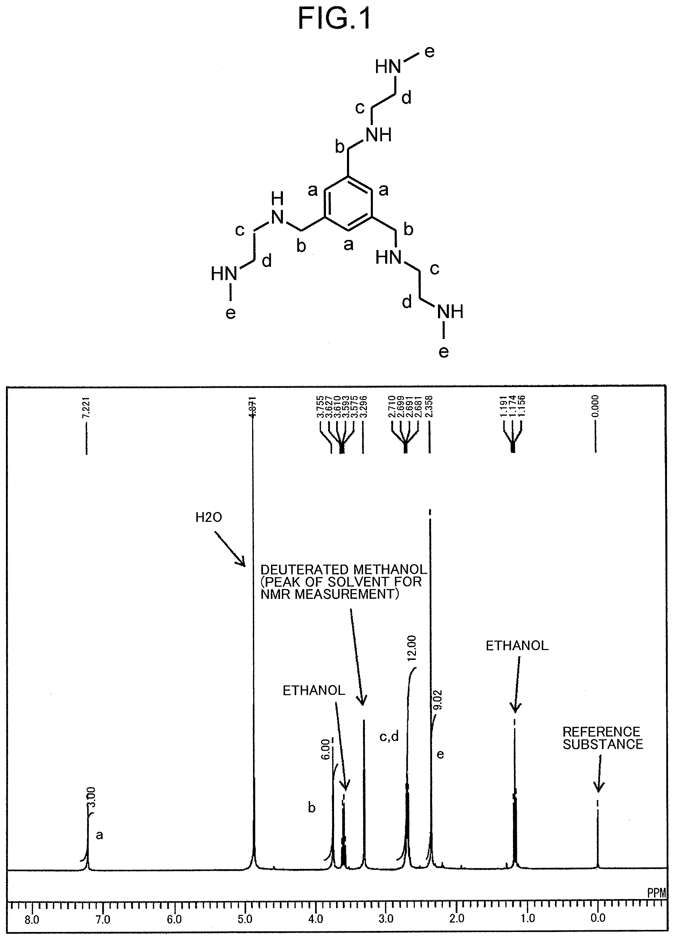

Whether or not the obtained substance was an amine having the structure represented by the above (1-1), which is the target product, was confirmed as follows.
1H-NMR: As a measurement solvent, deuterated ethanol was used. In FIG. 1 are shown a diagram showing existence positions a to e of hydrogen in the amine represented by the above (1-1), which is a target product of Example 1, and the measured 1H-NMR chart. In the 1H-NMR chart, which of a to e each peak corresponds to, and the number of hydrogen atoms in the peak are shown. As shown in FIG. 1, it has been confirmed from the 1H-NMR chart that the amine represented by the above (1-1) was produced.
GC and GCMS: The peak of the product was confirmed by GC, and mass spectrometry of the product was performed by GCMS to confirm the amine represented by the above (1-1).

### (Example 2)

In Example 2, an amine having a structure represented by the above (2-1), which was the amine of the first solid absorber, was used. The synthesis method is described in detail below.

First, isophthalaldehyde (reference substance) was dissolved in toluene, and an aqueous methylamine solution was added to the solution, followed by stirring at room temperature for 3 hours. After completion of the reaction, the toluene layer of the reaction solution was collected, and the solvent in the solution collected was evaporated and removed. Thereafter, the obtained intermediate substance was dissolved in acetone, and an imine was reduced with a reducing agent under further addition of methanol to the solution. Subsequently, a lipophilic component was collected from the reaction product by liquid-liquid extraction separation using chloroform and water, and from the collected lipophilic component, a target product supposed to be the amine having the structure represented by the above (2-1) was obtained as a brown oily substance. The yield was 83.3%.

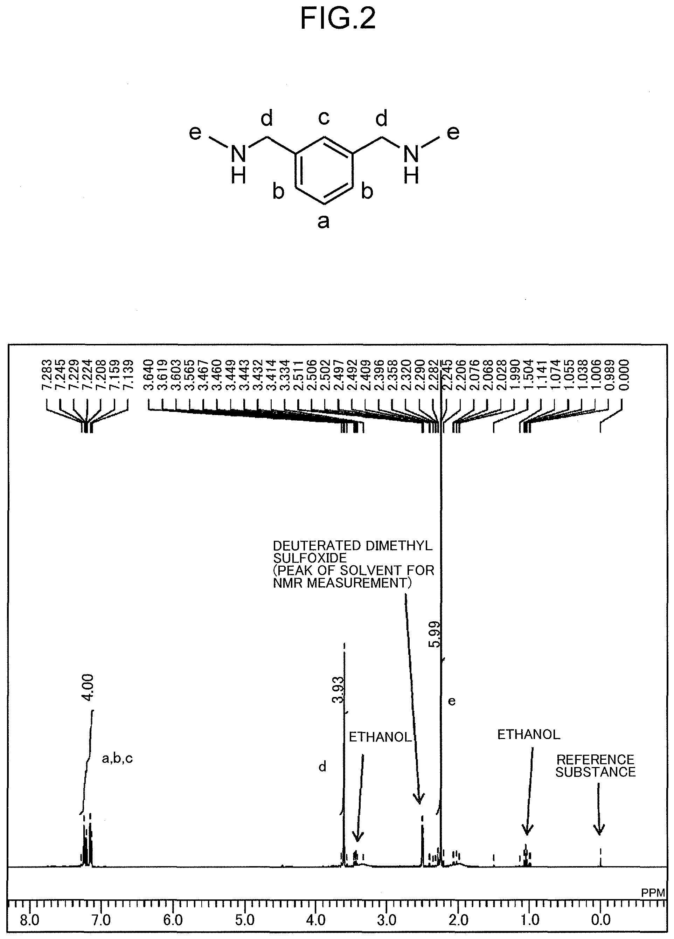

Whether or not the obtained substance was the amine having the structure represented by the above (2-1), which is the target product, was confirmed as follows.
1H-NMR: As a measurement solvent, deuterated dimethyl sulfoxide was used. In FIG. 2 are shown a diagram showing existence positions a to e of hydrogen in the amine represented by the above (2-1), which is a target product of Example 2, and the measured 1H-NMR chart. In the 1H-NMR chart, which of a to e each peak corresponds to, and the number of hydrogen atoms in the peak are shown. As shown in FIG. 2, it has been confirmed from the 1H-NMR chart that the amine represented by the above (2-1) was produced.
GC and GCMS: The peak of the product was confirmed by GC, and mass spectrometry of the product was performed by GCMS to confirm the amine represented by the above (2-1).

### (Example 3)

In Example 3, an amine having a structure represented by the above (3-1), which was the amine of the first solid absorber, was used. A target product supposed to be the amine having the structure represented by the above (3-1) was obtained by a synthesis method similar to that in Example 2 above except that terephthaldicarboxaldehyde (reference substance) was used as a starting substance instead of isophthalaldehyde. The yield was 67.9%.

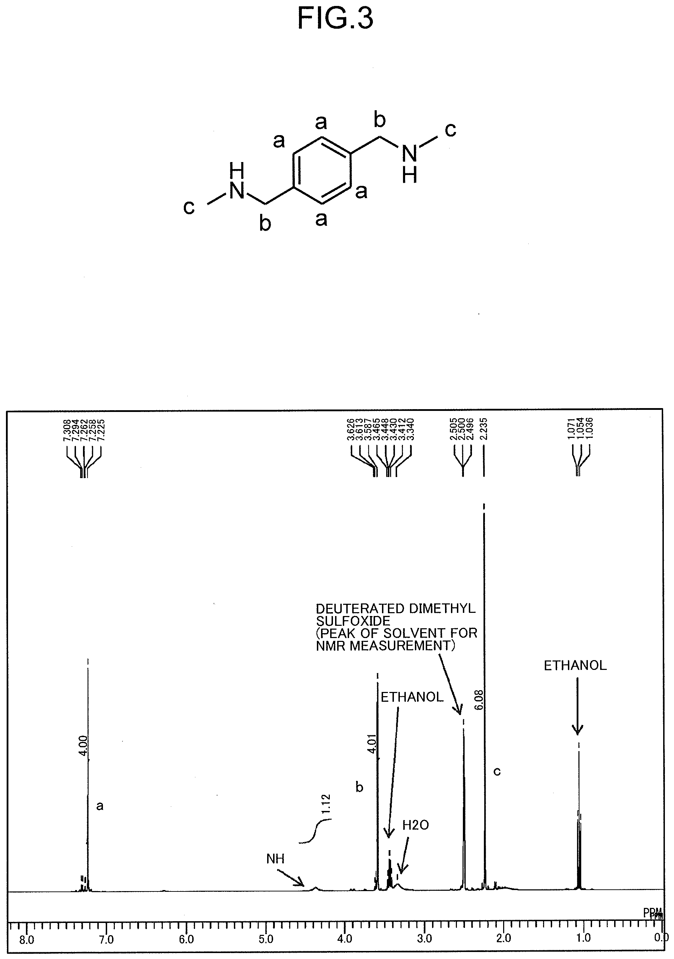

Whether or not the obtained substance was the amine having the structure represented by the above (3-1), which is the target product, was confirmed as follows.
1H-NMR: As a measurement solvent, deuterated dimethyl sulfoxide was used. In FIG. 3 are shown a diagram showing existence positions a to c of hydrogen in the amine represented by the above (3-1), which is a target product of Example 3, and the measured 1H-NMR chart. In the 1H-NMR chart, which of a to c each peak corresponds to, and the number of hydrogen atoms in the peak are shown. As shown in FIG. 3, it has been confirmed from the 1H-NMR chart that the amine represented by the above (3-1) was produced.
GC and GCMS: The peak of the product was confirmed by GC, and mass spectrometry of the product was performed by GCMS to confirm the amine represented by the above (3-1).

### (Example 4)

In Example 4, an amine having a structure represented by the above (4-4), which was the amine of the second solid absorber, was used. The synthesis method is described in detail below.

First, 3,3-diaminodipropylamine (reference substance), methyl ethyl ketone, and palladium carbon were charged into an autoclave, and ethanol was added thereto to homogenize the mixture. Hydrogen gas was then charged at 0.9 MPa into the mixture. Subsequently, the mixture was heated at 50°C for 90 hours. Thereafter, the reaction solution was filtered, and the solvent was removed to afford a target product supposed to be the amine having the structure represented by the above (4-4) as a yellow oily substance. The yield was 99.6%.

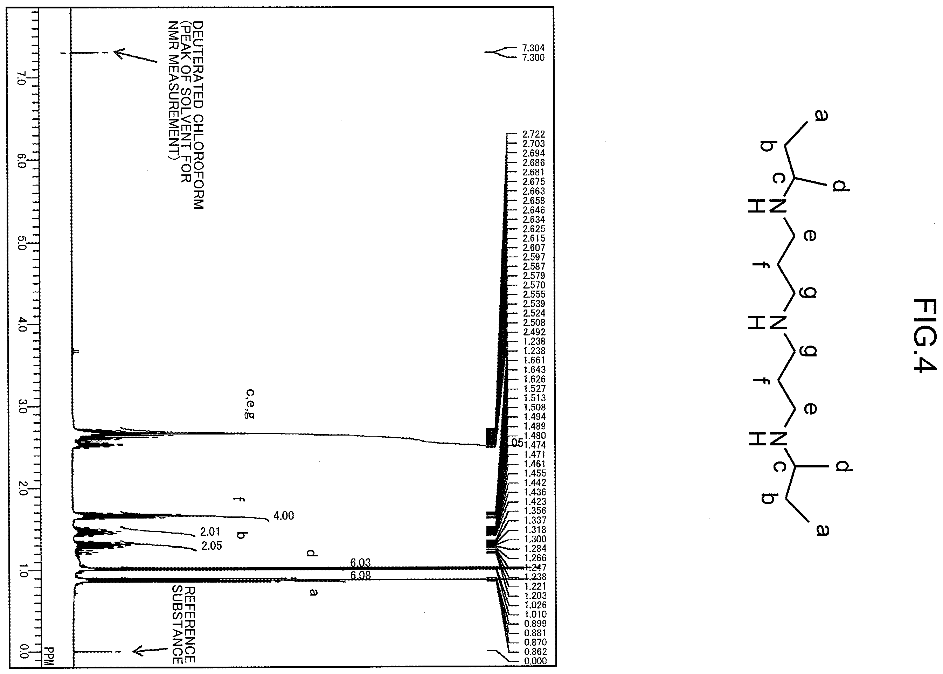

Whether or not the obtained substance was the amine having the structure represented by the above (4-4), which is the target product, was confirmed as follows.
1H-NMR: As a measurement solvent, deuterated chloroform was used. In FIG. 4 are shown a diagram showing existence positions a to g of hydrogen in the amine represented by the above (4-4), which is a target product of Example 4, and the measured 1H-NMR chart. In the 1H-NMR chart, which of a to g each peak corresponds to, and the number of hydrogen atoms in the peak are shown. As shown in FIG. 4, it has been confirmed from the 1H-NMR chart that the amine represented by the above (4-4) was produced.
GC and GCMS: The peak of the product was confirmed by GC, and mass spectrometry of the product was performed by GCMS to confirm the amine represented by the above (4-4).

### (Example 5)

In Example 5, an amine having a structure represented by the above (4-2), which was the amine of the second solid absorber, was used. The synthesis method was the same as that for the amine having the structure represented by the above (4-4), and a target product supposed to be the amine having the structure represented by the above (4-2) was obtained by the same procedure as that in Example 4 described above except that diethyl ketone was first used instead of methyl ethyl ketone. The yield was 73.5%.

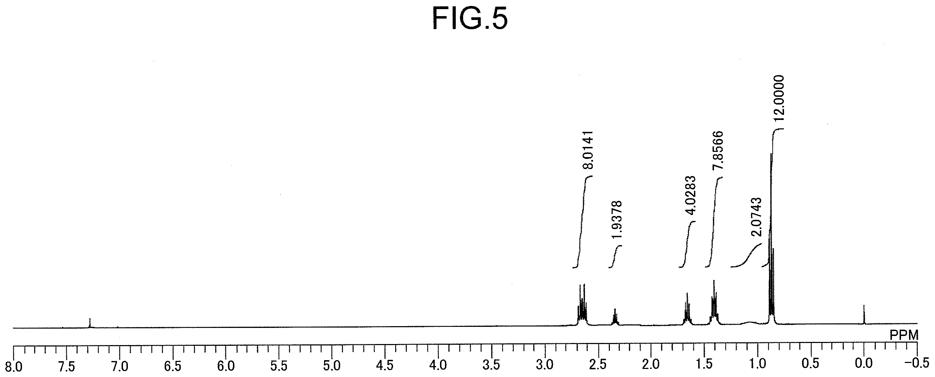

Whether or not the obtained substance was the amine having the structure represented by the above (4-2), which is the target product, was confirmed as follows.
1H-NMR: 1H-NMR was performed using a nuclear magnetic resonance apparatus ("BRUKER NMR 400 MHz" manufactured by Bruker Corporation). As a solvent, CDCl3 (20 to 40 mg/mL) was used. The measured 1H-NMR chart is shown in FIG. 5. From the 1H-NMR chart shown in FIG. 5, it has been confirmed that the amine represented by the above (4-2) was produced.

### (Example 6)

In Example 6, an amine having a structure represented by the above (4-3), which was the amine of the second solid absorber, was used. The synthesis method was the same as that for the amine having the structure represented by the above (4-4), and a target product supposed to be the amine having the structure represented by the above (4-3) was obtained by the same procedure as that in Example 4 described above except that methyl isobutyl ketone was first used instead of methyl ethyl ketone. The yield was 97.9%.

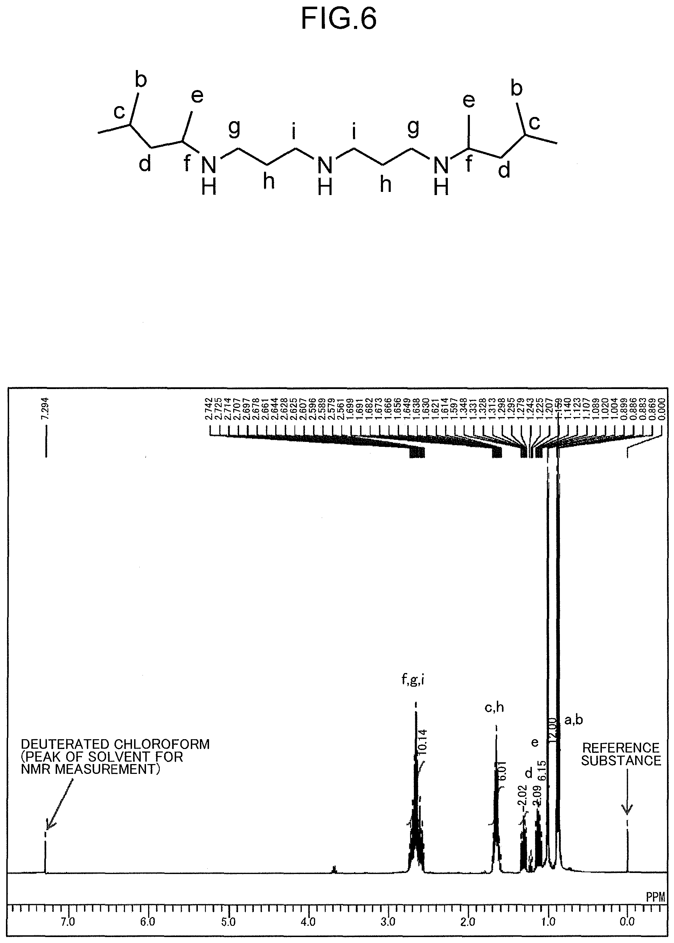

Whether or not the obtained substance was the amine having the structure represented by the above (4-3), which is the target product, was confirmed as follows.
1H-NMR: As a measurement solvent, deuterated chloroform was used. In FIG. 6 are shown a diagram showing existence positions a to i of hydrogen in the amine represented by the above (4-3), which is a target product of Example 6, and the measured 1H-NMR chart. In the 1H-NMR chart, which of a to i each peak corresponds to, and the number of hydrogen atoms in the peak are shown. As shown in FIG. 6, it has been confirmed from the 1H-NMR chart that the amine represented by the above (4-3) was produced.
GC and GCMS: The peak of the product was confirmed by GC, and mass spectrometry of the product was performed by GCMS to confirm the amine represented by the above (4-3).

### (Comparative Example 1)

In the comparative example, diethanolamine (DEA) (manufactured by FUJIFILM Wako Pure Chemical Corporation, boiling point: 217°C/760mmHg) was used as an amine to be carried on the porous base material. DEA is widely used as an amine that is a liquid chemical substance for reversibly absorbing carbon dioxide while being carried on a base material.

### [Porous Base Material]

As the porous base material, silica gel described in JP 6055134 B2 was used.

### [Method for Producing Solid Absorber]

First, about 300 mL of the porous base material was collected, and the mass thereof was measured. Subsequently, using water or ethanol as a solvent, an amine solution having a concentration of 45 mass% of one among the amines of Examples 1 to 6 synthesized above or DEA of Comparative Example was prepared, and the solution prepared was put into a bottle. The porous base material was charged thereinto, and the mixture was left to stand at room temperature for 8 hours or more with the porous base material entirely immersed in the liquid. Thereafter, the porous base material was taken out from the bottle, and subjected to centrifugation (1100 rpm, for 1 minute). After the centrifugation, the porous base material was filled in another container, and a drying gas (nitrogen gas at 40°C and 15 L/min) was caused to flow, whereby the porous base material carrying one of the amines of Examples 1 to 6 or DEA was through-flow dried. After a lapse of 2 hours from the time point at which the gas temperature at the outlet of the drying chamber for drying the porous base material carrying each amine or the like was stabilized, the drying was terminated, and the solvent was completely removed. In this way, porous base materials carrying the respective amines or DEA, namely, the solid absorbers in Examples 1 to 6 and Comparative Example were obtained.

The saturated carbon dioxide absorption amount and the saturated moisture absorption amount of the solid absorber of each of the Examples and Comparative Example produced by the above method were measured by the following methods.

### [Method for Measuring Saturated Carbon Dioxide Absorption Amount (mol/kg) and Saturated Moisture Absorption Amount (mol/kg) per Weight of Solid Absorber]

The saturated carbon dioxide absorption amount and the saturated moisture absorption amount per weight of the solid absorber in Examples 1 to 6 and Comparative Example were measured by the following methods. First, a cylindrical container having a volume of 150 mL was filled with a solid absorber produced by the above-described method. Subsequently, carbon dioxide or moisture (H₂O) having been absorbed previously by the solid absorber was desorbed by feeding nitrogen gas at 40°C into the cylindrical container at 50 L/min. Thereafter, the initial weight of the container filled with the solid absorber was measured, and then a gas prepared to have a prescribed composition containing carbon dioxide or moisture was fed into the container at 50 L/min. Thereafter, the weight of the container was measured at regular time intervals, and it was determined that the absorption of carbon dioxide or the absorption of moisture had reached saturation at the time when almost no change in weight was detected. Then, the saturated carbon dioxide absorption amount (mol/kg) per weight of the solid absorber or the saturated moisture absorption amount (mol/kg) per weight of the solid absorber was calculated from the weight change of the container between the initial stage and the saturation. When the saturated carbon dioxide absorption amount was measured, a gas obtained by mixing carbon dioxide and nitrogen gas was used. On the other hand, when the saturated moisture absorption amount was measured, a gas whose humidity was adjusted by allowing nitrogen gas to permeate water was used.

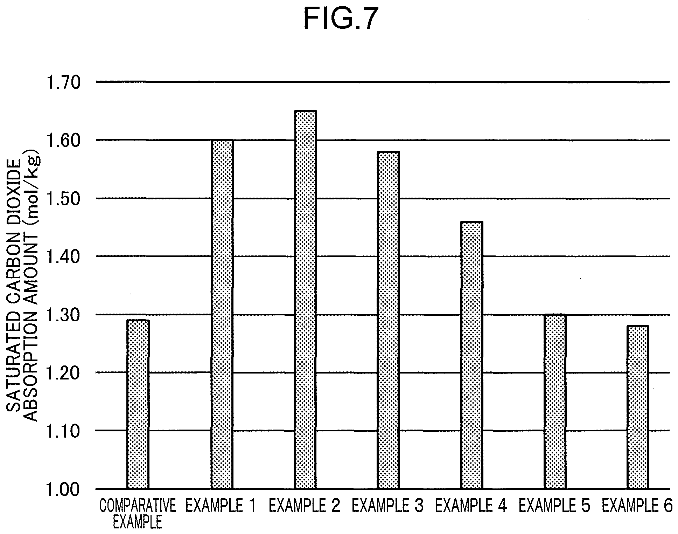

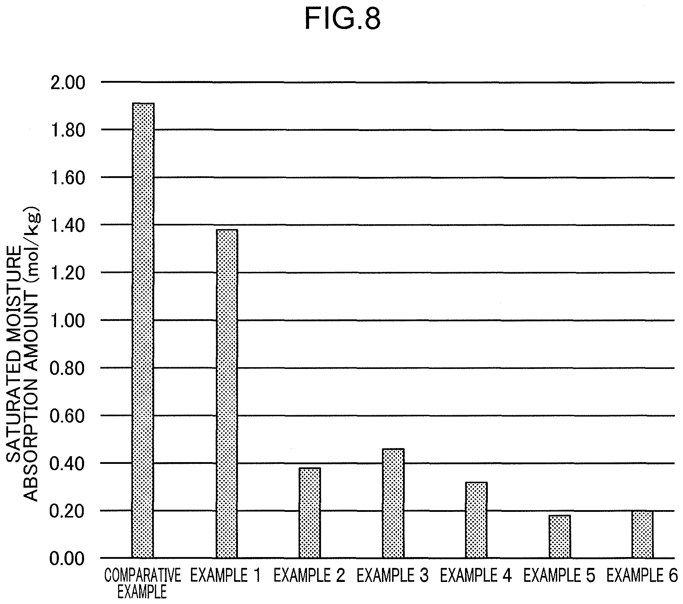

The measurement results of the saturated carbon dioxide absorption amount and the saturated moisture absorption amount per weight of the solid absorber of each of Examples and Comparative Example are shown in the graphs of FIGS. 7 and 8.

As shown in FIG. 7, the solid absorbers of Examples 1 to 6, which are the first or second solid absorbers in the present embodiment, were able to absorb carbon dioxide equally to or more than the solid absorber of Comparative Example. In addition, as shown in FIG. 8, the solid absorbers of Examples 1 to 6 had significant low-hygroscopicity as compared with the solid absorber of Comparative Example. That is, the solid absorbers of Examples 1 to 6 have better ease in handling as compared with the solid absorber of Comparative Example because of their low-hygroscopicity, and thus can desorb carbon dioxide efficiently. From these results, it has been found that the first and second solid absorbers in the present embodiment have low hygroscopicity and are capable of efficiently and reversibly absorbing carbon dioxide.

This application is based on Japanese Patent Application No. 2023-200852 filed on November 28, 2023, the contents of which are included in the present application.

It should be understood that the embodiments and examples disclosed herein are illustrative and not restrictive in all respects. The scope of the present invention is defined not by the above description but by the claims, and it is intended to encompass all modifications within the meanings and scope that are equivalent to the claims.

## Claims

1. A solid absorber that reversibly absorbs an acidic gas,
the solid absorber comprising a porous base material and an acidic gas absorbing agent carried on the porous base material
wherein the acidic gas absorbing agent comprises at least one amine represented by any one of the following general formulae (1), (2), and (3): 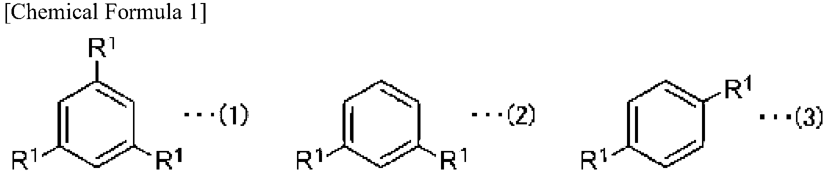
in the formulae (1), (2), and (3), R¹ is represented by group: -(CH₂)ₗ-NH-[(CH₂)₂NH]ₘ-(CH₂)ₙ-CH₃, and in R¹, l represents 1 or 2, and m and n each independently represent an integer of 0 to 2.

2. The solid absorber according to claim 1, wherein the amine has a structure represented by any one of the following (1-1), (1-2), (2-1), and (3-1).

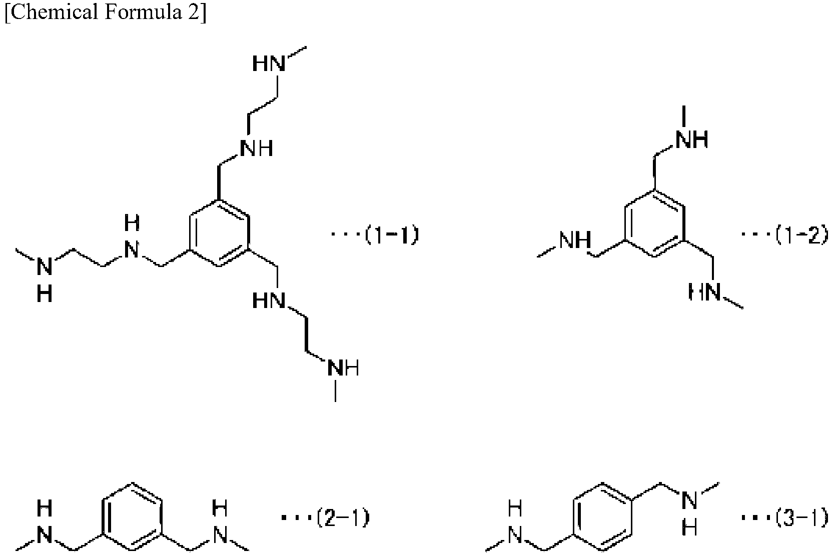

3. A solid absorber that reversibly absorbs an acidic gas,
the solid absorber comprising a porous base material and an acidic gas absorbing agent carried on the porous base material
wherein the acidic gas absorbing agent comprises at least one amine represented by the following general formula (4): 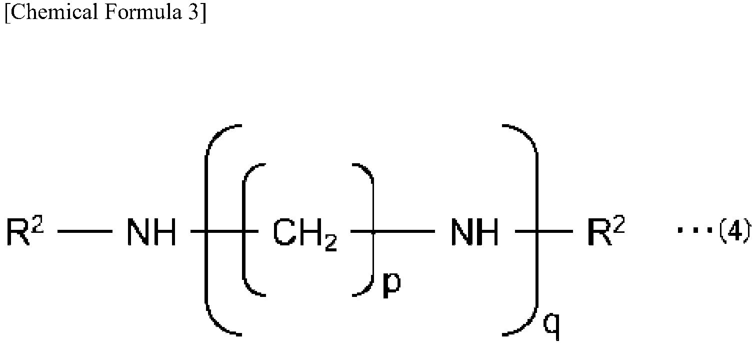
in the formula (4), two R² groups may have the same structure or different structures, p represents 2 or 3, q represents an integer of 2 to 5, and R² is a group represented by any one of the following R²(1) to R²(9).

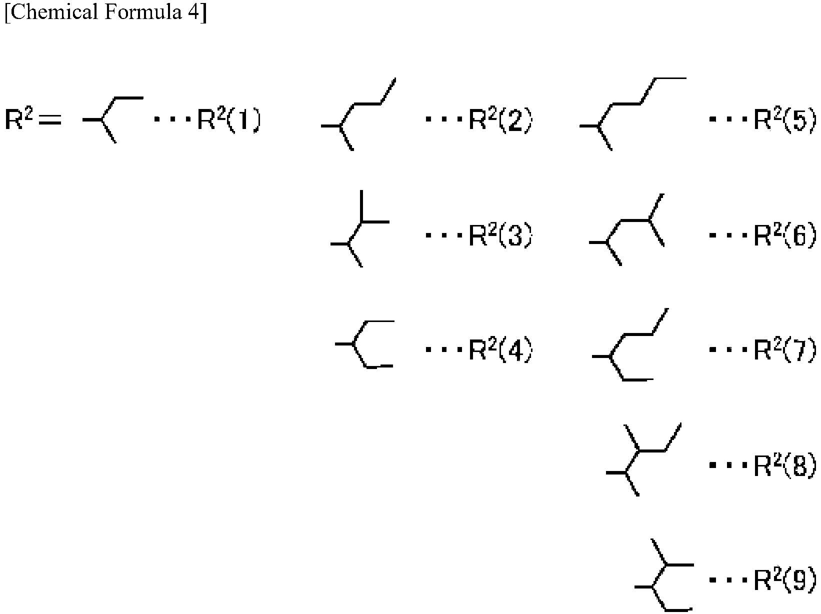

4. The solid absorber according to claim 3, wherein the amine has a structure represented by any one of the following (4-1) to (4-6).

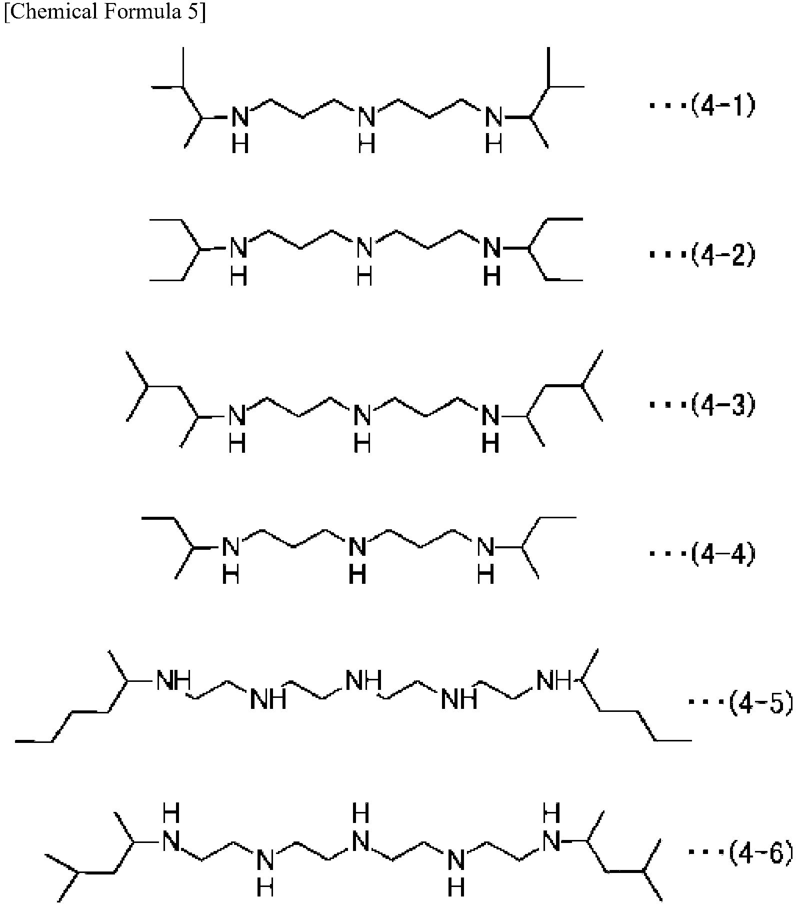

5. A method for absorbing and desorbing an acidic gas comprising:
a step of bringing a gas to be treated into contact with the solid absorber according to any one of claims 1 to 4 to cause the solid absorber to absorb the acidic gas, and
a step of bringing steam into contact with the solid absorber having absorbed the acidic gas to cause the acidic gas to be desorbed from the solid absorber.
